(19) Europäisches Patentamt
European Patent Office
Office européen des brevets

(11) **EP 0 900 368 B1**

(12) **EUROPÄISCHE PATENTSCHRIFT**

(45) Veröffentlichungstag und Bekanntmachung des Hinweises auf die Patenterteilung:
**22.09.2004 Patentblatt 2004/39**

(51) Int Cl.⁷: **G01N 3/40**, G01N 11/16
// G01N33/44

(21) Anmeldenummer: **98912360.9**

(22) Anmeldetag: **20.02.1998**

(86) Internationale Anmeldenummer:
**PCT/EP1998/001003**

(87) Internationale Veröffentlichungsnummer:
**WO 1998/037401 (27.08.1998 Gazette 1998/34)**

(54) **VORRICHTUNG ZUR MESSUNG VISKOELASTISCHER EIGENSCHAFTEN VON KÖRPERN**

DEVICE FOR MEASURING THE ELASTIC-VISCOUS PROPERTIES OF OBJECTS

DISPOSITIF DE MESURE DES PROPRIETES VISCOELASTIQUES D'OBJETS

(84) Benannte Vertragsstaaten:
**AT BE CH DK ES FI FR GB GR IE IT LI LU NL PT SE**

(30) Priorität: **20.02.1997 DE 19706744**

(43) Veröffentlichungstag der Anmeldung:
**10.03.1999 Patentblatt 1999/10**

(73) Patentinhaber: **Dunlop GmbH & Co. KG**
**63450 Hanau (DE)**

(72) Erfinder:
• **JAKOB, Karlheinz**
**D-63571 Gelnhausen (DE)**
• **UNSELD, Klaus**
**D-63450 Hanau (DE)**
• **HERRMANN, Volker**
**D-97070 Würzburg (DE)**

• **FUCHS, Hans-Bernd**
**D-63755 Alzenau (DE)**

(74) Vertreter: **Kutsch, Bernd**
**Goodyear S.A.**
**Patent Department**
**7750 Colmar-Berg (LU)**

(56) Entgegenhaltungen:
**EP-A- 0 466 060          EP-A- 0 764 842**
**DE-A- 4 040 786          US-A- 4 383 450**
**US-A- 4 450 713**

• **PATENT ABSTRACTS OF JAPAN vol. 096, no. 012, 26.Dezember 1996 & JP 08 201259 A (AKASHI:KK), 9.August 1996,**

**Beschreibung**

Die vorliegende Erfindung betrifft eine Vorrichtung zur Messung viskoelastischer Eigenschaften von Körpern, insbesondere von Körpern aus elastomerem Material wie Gummi, mit einem gegenüber einer Halterung verschiebbar gelagerten Meßkörper mit einem auf einem Probekörper aufsetzbaren Meßkopf, Mitteln zum Hin- und Herbewegen des Meßkörpers und Mitteln zum Messen des Verschiebeweges des Meßkopfes und/oder der auf den Meßkopf wirkenden Kraft während des Hin- und Herbewegens des Meßkörpers.

Eine derartige Vorrichtung ist aus der DE 40 40 786 A1 bekannt. Bei dieser bekannten Vorrichtung wird ein Probekörper zur Messung auf einen Meßtisch gelegt, der unterhalb der Halterung mit dem Meßkörper vorgesehen ist. Die Halterung muß dabei gegenüber dem Meßtisch starr abgestützt sein, um bei der Messung reproduzierbare Ergebnisse zu erzielen.

Diese Vorrichtung hat zwar bereits gegenüber früheren Vorrichtungen zum Messen viskoelastischer Eigenschaften den Vorteil, daß keine spezielle Probenvorbereitung und insbesondere kein Einspannen der Probe in eine Einspannvorrichtung erforderlich ist. Dennoch weist diese Vorrichtung Nachteile bei der Einsetzbarkeit auf, da die Proben auf dem Probentisch unterhalb des Meßkörpers angeordnet werden müssen. Die Anordnung des Probekörpers auf dem Probentisch benötigt Zeit und ist nicht bei allen Probekörpern möglich, insbesondere wenn diese keine einigermaßen ebene Auflagefläche aufweisen.

Der Erfindung liegt die Aufgabe zugrunde, eine Vorrichtung der eingangs genannten Art so weiterzubilden, daß diese Nachteile nicht auftreten. Insbesondere sollen die Einsatzmöglichkeiten vergrößert und die Handhabung vereinfacht werden.

Diese Aufgabe wird dadurch gelöst, daß der Meßkörper in einem frei beweglichen Gehäuse mit einer Öffnung für den Durchtritt des Meßkopfes und einer auf dem Probekörper aufsetzbaren Anschlagfläche gelagert ist.

Im Gegensatz zu der bekannten Vorrichtung wird erfindungsgemäß also nicht der Probekörper in eine bestimmte Lage zur Vorrichtung gebracht, sondern das frei bewegliche Gehäuse der erfindungsgemäßen Vorrichtung wird zur Messung der Viskoelastizität auf den Probekörper aufgesetzt. Die Anschlagfläche gewährleistet dabei, daß der Probekörper eine definierte Lage zum Gehäuse mit dem Meßkörper einnimmt. Dadurch ist es möglich, auf einen gegenüber der Halterung des Meßkörpers starren Probenauflagetisch zu verzichten und direkt an der Probe zu messen. Die erfindungsgemäße Vorrichtung kann daher auch als Handgerät ausgebildet werden, welches direkt vor Ort einsetzbar ist.

Die Messung viskoelastischer Eigenschaften ist unter anderem bei Fahrzeugreifen von besonderer Wichtigkeit, beispielsweise bei der Erprobung neuer Reifenmischungen. Bisher wurden hierfür Probekörper aus dem jeweiligen Reifenmaterial hergestellt und in eine Meßvorrichtung gemäß DIN 53513 fest eingespannt. Nachteilig ist hierbei, daß zuerst Probekörper hergestellt oder aus einem vorhandenen Reifen ausgeschnitten werden müssen, bevor eine Messung stattfinden kann. Dies hat zudem den Nachteil, daß ein Reifen nach erfolgter Messung zerstört ist. Damit ist es auch nicht möglich, ein und denselben Reifen nach unterschiedlicher Laufleistung zu messen.

Die Erfindung ermöglicht es nun, die Viskoelastizität verschiedener Reifenbestandteile wie beispielsweise Lauffläche und Seitenwand zu messen, ohne den Reifen zu zerstören. Hierfür wird das Gehäuse mit der Anschlagfläche auf eine geeignete Stelle des Fahrzeugreifens aufgesetzt und die Messung ausgeführt. Nach beendeter Messung kann der Reifen wieder benutzt werden, beispielsweise um nach einer höheren Laufleistung eine erneute Messung der Viskoelastizität oder auch Messungen anderer Art auszuführen. Ebenso ist es mit dem erfindungsgemäßen Handgerät nun möglich, Messungen an einem auf ein Fahrzeug aufgezogenen Reifen vorzunehmen. Eine Messung kann also beispielsweise unmittelbar nach Abschluß einer Probefahrt an dem noch heißen Reifen durchgeführt werden, wodurch eine weit einfachere, schnellere und wirklichkeitsnähere Messung der Viskoelastizität eines erhitzten Reifens möglich ist als bei einem unabhängig erhitzten Probekörper aus Reifenmaterial.

Nach einer Ausgestaltung der Erfindung sind Mittel vorgesehen, durch welche sicherstellbar ist, daß die Anschlagfläche des Gehäuses während des Meßvorgangs fest auf dem Probekörper aufsitzt. Durch dieses Merkmal wird die Verwendung der erfindungsgemäßen Vorrichtung als Handgerät verbessert, indem verhindert wird, daß sich das Gehäuse mit dem Meßkörper während des Meßvorganges relativ zu dem Probekörper bewegt. Dadurch ist gewährleistet, daß auch mit einem Handgerät reproduzierbare Ergebnisse hoher Qualität erreicht werden können.

Eine Möglichkeit für ein solches Mittel besteht darin, die Anschlagfläche als Standfläche definierter Größe auszubilden. Das Gehäuse mit dem Meßkörper kann dann mit seiner Standfläche auf den Probekörper gestellt werden, so daß der feste Kontakt zwischen der Anschlagfläche und dem Probekörper durch das Eigengewicht des Gehäuses gewährleistet ist. Das Gehäuse kann dabei beispielsweise ein Gewicht zwischen circa 0,5 und 1 kp aufweisen.

Eine andere Möglichkeit besteht darin, einen Drucksensor vorzusehen, welcher ein Anpressen der Anschlagfläche an dem Probekörper detektiert. Ein solcher Drucksensor kann beispielsweise durch eine zwischen zwei einander zugewandte ebene Flächen zweier Teile des Gehäuses eingespannte ebene Druckscheibe gebildet sein. Eine gültige Messung liegt nur dann vor, wenn der Drucksensor einen Anpreßdruck oberhalb eines bestimmten Schwellwertes detektiert.

Die Auswahl gültiger Messungen kann dabei entweder über entsprechende automatische Mittel erfolgen, oder aber manuell einfach dadurch, daß eine geeignete Anzeigevorrichtung, beispielsweise eine Lampe, anzeigt, wenn ein ausreichender Anpreßdruck des Gehäuses am Probekörper gegeben ist.

Nach einer weiteren Ausgestaltung der Erfindung ist die Nullstellung des Meßkopfes durch eine vorbestimmte Eindringtiefe festgelegt. Anders als bei der aus der DE 40 40 786 A bekannten Vorrichtung wird die Nullstellung also nicht über die Eindrückkraft des Meßkopfes in den Probekörper festgelegt. Dies hat insbesondere bei verhältnismäßig weichen Probekörpern den Vorteil, daß ein zu tiefes Eindringen des Meßkopfes und damit die Gefahr einer Zerstörung des Probekörpers vermieden wird. Dies ist unter anderem auch deshalb wichtig, weil die erfindungsgemäße Vorrichtung auch an einem Endprodukt vor Ort und nicht nur an einem speziell hergestellten Probekörper eingesetzt werden soll.

Bevorzugt ist die Eindringtiefe in Nullstellung so gewählt, daß die Auslenkung des Meßkopfes während der Messung in einem Bereich zumindest annähernd linearen Kraft-Weg-Verhältnisses liegt. Hierdurch wird die Aussagekraft der erhaltenen Meßwerte erhöht. Bei Messung eines Körpers aus elastomerem Material beträgt die Eindringtiefe in Nullstellung beispielsweise circa. 100 bis ca. 500 µm, bei Messung von Reifenmischungen, insbesondere circa 500 µm. Diese Werte können jedoch, insbesondere in Abhängigkeit von der Form des Eindringkörpers, auch anders gewählt werden. Vorteilhaft ist es, geeignete Werte durch Voruntersuchungen zu ermitteln.

Nach einer weiteren Ausgestaltung der Erfindung steht der Meßkopf entsprechend der gewünschten Eindringtiefe über die Anschlagfläche des Gehäuses über. Durch Aufsetzen des Gehäuses mit der Anschlagfläche auf den Probekörper wird damit auf einfache Weise das Eindringen des Meßkopfes in den Probekörper um den gewünschten Betrag erreicht. Eine andere Möglichkeit besteht darin, Mittel vorzusehen, durch welche der Meßkopf aus dem Gehäuse in seine Nullstellung ausfahrbar ist, bevor die Messung begonnen wird. In beiden Fällen ist die Nullstellung des Meßkopfes bevorzugt wählbar. Die Nullstellung kann also an dem Gerät, insbesondere in Abhängigkeit von dem Material des Probekörpers, in geeigneter Weise eingestellt werden.

Nach einer weiteren Ausgestaltung der Erfindung ist die Auslenkung des Meßkopfes während der Messung durch den Auslenkweg festgelegt. Wiederum im Gegensatz zu der DE 40 40 786 A wird die Auslenkung also nicht durch die Auslenkkraft bestimmt. Auch hierdurch kann eine möglicherweise zu starke Belastung und damit die Gefahr einer Zerstörung des Probekörpers vermieden werden.

Ebenso ist es auch hier bevorzugt, wenn der Auslenkweg an dem Gerät wählbar ausgestaltet ist. Die Auslenkung kann dadurch einerseits in Abhängigkeit von dem Material des Probekörpers geeignet gewählt werden. Andererseits besteht die Möglichkeit, an ein und demselben Probekörper Messungen mit verschiedener Auslenkung vorzunehmen und dadurch zusätzliche Aussagen über den Probekörper zu erhalten.

Der Auslenkweg ist nach einer weiteren Ausgestaltung der Erfindung bevorzugt möglichst klein gewählt. Dadurch wird nicht nur die Gefahr einer Zerstörung des Probekörpers verringert, sondern auch die Qualität der erhaltenen Ergebnisse verbessert, da sich der Probekörper bei kleinen Auslenkungen annähernd linear verhält. Es hat sich gezeigt, daß hierdurch Meßwerte erhalten werden, die sehr nahe an herkömmlichen Meßgeräten mit fest eingespannten Probekörpern liegen. Der Auslenkweg zur Messung eines Körpers aus elastomerem Material beträgt beispielsweise circa 5 bis 25 µm, bei einem Probekörper aus Reifenmaterial insbesondere circa 5 µm.

Nach einer weiteren Ausgestaltung der Erfindung ist auch die Frequenz der Hin- und Herbewegung des Meßkopfes wählbar. Auch in diesem Fall kann die Frequenz sowohl in Abhängigkeit von dem Material des Probekörpers als auch zur Erlangung zusätzlicher Aussagen über den Probekörper variiert werden. Bei einem Probekörper aus elastomerem Material beträgt die Frequenz bevorzugt zwischen circa 1 bis 15 Hz, insbesondere circa 10 Hz. Diese Frequenzen haben sich insbesondere zur Messung von Probekörpern aus Reifenmaterial als besonders geeignet herausgestellt. Die Anregung kann insbesondere nach einer Sinusfunktion oder einer anderen periodischen Funktion, beispielsweise Rechtecks- oder Dreiecksfunktion, erfolgen.

Nach einer weiteren Ausgestaltung der Erfindung sind Mittel zur Bestimmung der Temperatur des Probekörpers vorgesehen. Besonders geeignet sind hierbei berührungslos arbeitende Temperatursensoren wie beispielsweise Infrarotsensoren. Durch Messung der Temperatur des Probekörpers kann einerseits gewährleistet werden, daß die erhaltenen Meßwerte reproduzierbar und untereinander vergleichbar sind. Andererseits können hierdurch zusätzliche Aussagen über das Verhalten des Probekörpers erhalten werden, so kann beispielsweise die Viskoelastizität von Reifenmaterial bei verschiedenen Betriebstemperaturen eines Fahrzeugreifens ermittelt werden.

Zur Bewegung des Meßkopfes in dem Gehäuse ist bevorzugt ein Piezoelement, insbesondere ein sogenannter Stapeltranslator vorgesehen. Ein Piezoelement ermöglicht vorteilhafterweise eine Auslenkung des Meßkopfes um geringe Werte, wie es zur Erreichung eines linearen Verhaltens des Probekörpers wünschenswert ist. Bevorzugt ist es dabei, wenn das Piezoelement mit einer Wegmeßeinrichtung in einem Regelkreis angeordnet ist. Hierdurch kann gewährleistet werden, daß der vorgesehene Auslenkweg des Meßkörpers exakt eingehalten wird. Als Wegmeßeinrichtung kann beispielsweise ein weiteres Piezoelement oder ein Dehnungsmeßstreifen dienen.

Nach einer weiteren Ausgestaltung der Erfindung ist der Meßkopf als Eindringkörper gemäß DIN 53505 ausgebildet, zur Messung von Probekörpern aus elastomerem Material insbesondere als Shore-A-Eindringkörper. Durch Verwendung eines DIN-Eindringkörpers können mit der erfindurigsgemäßen Vorrichtung Meßwerte erhalten werden, die unmittelbar mit DIN-Meßwerten vergleichbar sind. Da die mit der erfindungsgemäßen Vorrichtung erhaltenen Meßwerte eine sehr hohe Qualität aufweisen, kann in vielen Fällen auf eine DIN-Messung verzichtet werden. Selbstverständlich können aber auch andere Eindringkörper verwendet werden, wobei geeignete Meßbedingungen, insbesondere Eindringtiefe, aber auch Frequenz und andere, jeweils bevorzugt durch Voruntersuchungen, insbesondere in Abhängigkeit vom Eindringkörper und vom untersuchten Material, ermittelt werden.

Die erfindungsgemäße Vorrichtung kann in vielfältiger Weise eingesetzt werden. So kann sie bereits während der Entwicklung von Reifenmischungen eingesetzt werden, wobei sogar eine Messung an noch unvulkanisiertem Material gute Ergebnisse liefert. Außerdem ist eine Messung am fertigen Reifen und an einem montierten Reifen möglich.

Ein Ausführungsbeispiel der Erfindung ist in der Zeichnung dargestellt und wird nachfolgend beschrieben. Es zeigen, jeweils in schematischer Darstellung,

Fig. 1      einen Schnitt durch das Gehäuse mit Meßkörper einer erfindungsgemäßen Vorrichtung,

Fig. 2      eine spezifizierte Darstellung des unteren Abschnitts des Meßkörpers der Vorrichtung von Fig. 1 und

Fig. 3      eine mögliche Gesamtanordnung einer erfindungsgemäßen Vorrichtung.

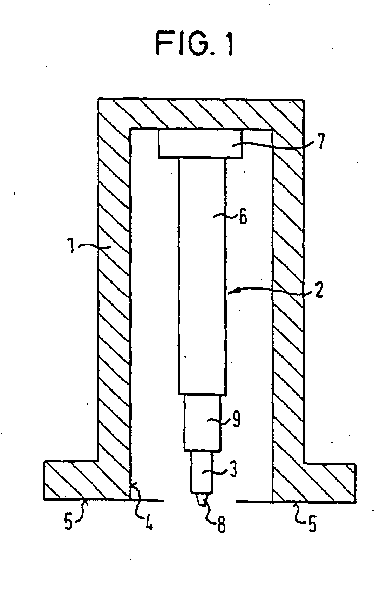

Wie in Fig. 1 dargestellt, ist in einem Gehäuse 1 ein Meßkörper 2 mit einem Meßkopf 3 angeordnet. Das Gehäuse 1 weist eine Öffnung 4 auf, durch welche der Meßkopf 3 des Meßkörpers 2 aus dem Gehäuse 1 hervorragt, sowie eine Anschlagfläche 5, mit welcher das Gehäuse 1 auf einem Probekörper aufsetzbar ist. Die Anschlagfläche 5 ist dabei um die Öffnung 4 angeordnet, so daß der herausragende Teil des Meßkopfes 3 zugleich den Überstand a des Meßkopfes 3 über die Anschlagfläche 5 bestimmt.

Der Meßkörper 2 umfaßt ein als Stapeltranslator ausgebildetes Piezoelement 6, welches mit einem durch einen Quarzkristall gebildeten Kraftaüfnehmer 7 verbunden ist, der seinerseits an dem Gehäuse 1 befestigt ist. Der Meßkopf 3 des Meßkörpers 2 weist eine als Shore-A-Eindringkörper gemäß DIN 53505 ausgebildete Spitze 8 auf und ist mit dem unteren Ende des Piezoelementes 6 über ein Verbindungselement 9 verbunden.

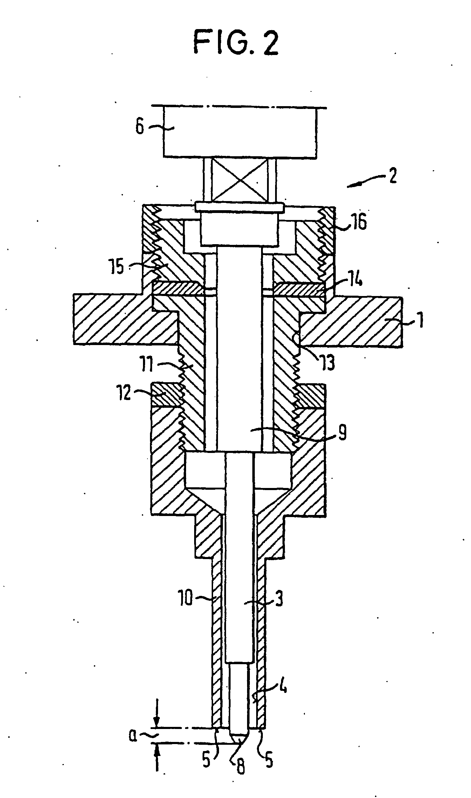

In Fig. 2 ist eine bevorzugte Ausgestaltung des unteren Abschnitts des Meßkörpers 2 genauer erkennbar. Der Meßkopf 3 ist von einer Anschlaghülse 10 umgeben, an deren unterem Ende die Öffnung 4 und die Anschlagfläche 5 ausgebildet sind. Die Anschlaghülse 10 ist auf eine Druckhülse 11 aufgeschraubt und mittels einer Kontermutter 12 gekontert. Die Druckhülse 11 ist in eine Ausnehmung 13 des Gehäuses 1 eingesetzt und unter Zwischenschaltung einer ebenen Druckscheibe 14 mittels einer mit dem Gehäuse 1 verschraubten Einschraubhülse 15 und einer Kontermutter 16 dort fixiert. Die Druckhülse 11 ist somit in der Ausnehmung 13 gegenüber der fest am Gehäuse 1 abgestützten Druckscheibe 14 beweglich und überträgt die über die Anschlagfläche 5 auf die Anschlaghülse 10 ausgeübten Kräfte auf die Druckscheibe 14. Nicht dargestellt sind Mittel zur Erzeugung eines Signals in Abhängigkeit von der Größe des auf die Druckscheibe 14 ausgeübten Druckes.

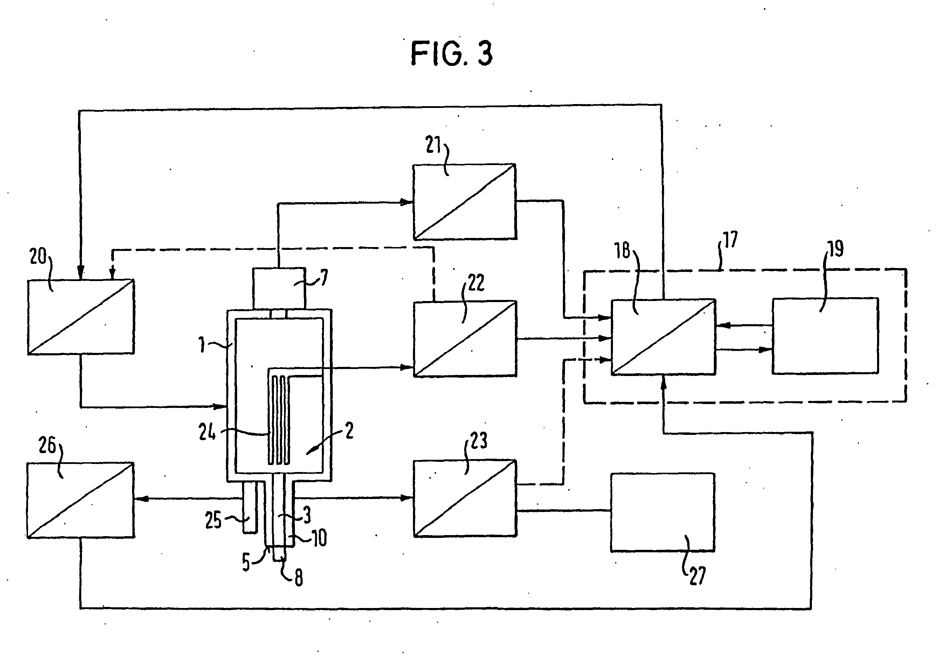

Die in Fig. 3 gezeigte Prinzipdarstellung einer möglichen Gesamtanordnung einer erfindungsgemäßen Vorrichtung umfaßt neben dem Gehäuse 1 mit dem Meßkörper 2 eine Auswerte- und Steuereinheit 17, beispielsweise einen mit einem Analog/Digitalwandler 18 ausgerüsteten Rechner 19. Die Auswerte- und Steuereinheit 17 ist über einen Meßumformer 20 mit dem Meßkörper 2 verbunden. Außerdem erhält die Auswerte- und Steuereinheit 17 über jeweils einen Meßumformer 21, 22 und 23 Ausgangssignale des Kraftaufnehmers 7, einer beispielsweise als Dehnungsmeßstreifen ausgebildeten Wegmeßeinrichtung 24 und des Drucksensors 14. An dem Gehäuse 1 ist zudem eine insbesondere als Infrarotsensor ausgebildete Temperaturmeßeinrichtung 25 angeordnet, deren Ausgangssignale über einen weiteren Meßumformer 26 zugeführt werden. Zusätzlich oder alternativ können die Ausgangssignale der Temperaturmeßeinrichtung 25, wie dargestellt, der Auswerte- und Steuereinheit 17 zuführbar sein.

Zur Messung der viskoelastischen Eigenschaften eines Probekörpers mit der erfindungsgemäßen Vorrichtung wird das Gehäuse 1 auf den Probekörper derart aufgesetzt, daß die Spitze 8 des Meßkopfes 3 in den Probekörper eindringt und die Anschlagfläche 5 der Anschlaghülse 10 auf dem Probekörper aufsitzt. Über den Drucksensor 14 wird festgestellt, ob ein ausreichender Anpreßdruck der Anpreßhülse 10 auf dem Probekörper gegeben ist, um eine stabile Messung zu gewährleisten. Bei Überschreiten eines entsprechend voreingestellten Schwellwertes wird die Anzeige 27 mit einem Ausgangssignal des Drucksensors 14 beaufschlagt. Dadurch wird angezeigt, daß eine verwertbare Messung möglich ist. Zusätzlich oder alternativ kann das Ausgangssignal des Drucksensors 14 der Auswerteund Steuereinheit 17 zugeführt werden, um eine Verwendung eines erhaltenen Meßwertes nur bei ausreichendem Anpreßdruck der Anpreßhülse 10 auf den Probekörper zu ermöglichen.

Bei auf den Probekörper aufgesetztem Gehäuse 1 wird die Viskoelastizitätsmessung durchgeführt, indem die Auswerte- und Steuereinheit 17 über den Meßumformer 20 den Meßkörper 2 zu Schwingungen einer gewünschten Frequenz veranlaßt. Die Bewegung des Meßkörpers 2 wird dabei durch das Piezoelement 6 bewirkt und einerseits durch den Kraftaufnehmer 7 und andererseits die Wegmeßeinrichtung 24 detektiert. Zugleich kann über die Temperaturmeßeinrichtung 25 die Temperatur des Probekörpers ermittelt werden. Die Meßsignale des Kraftaufnehmers 7, der Wegmeßeinrichtung 24 und der Temperaturmeßeinrichtung 25 werden über die Meßumformer 21, 22 und 26 der Auswerte- und Steuereinheit 17 zugeführt. Über den Analog/Digitalwandler 18 werden die Daten dem Rechner 19 zugeführt, wo sie angezeigt und einer weiteren Bearbeitung unterworfen werden können.

Aus den erhaltenen Daten wird in bekannter Weise, beispielsweise nach der Formel:

$$\sigma(t) = E[\varepsilon_m + \varepsilon_a \cdot \sin(\omega t)] + \eta\omega\varepsilon_a \cdot \cos(\omega t)$$

oder der Formel:

$$f(t) = S_H[\ell_o + \ell_a \cdot \sin(\omega t)] + \eta_H\omega\ell_a \cdot \cos(\omega t),$$

die Viskoelastizität des Probekörpers bestimmt, insbesondere als Real- und Imaginärteil der Steifigkeit $S_H$ als eine dem Elastizitätsmodul verwandte Größe. Hierbei ist

$\sigma(t)$ Spannungsverlauf über der Zeit in MPa

$E$ Elastizitätsmodul in MPa

$\eta$ Viskosität in MPa·s

$\varepsilon_m$ statische Dehnung in %

$\varepsilon_a$ Dehnungsamplitude in %.

$\omega$ Kreisfrequenz in Hz

$f(t)$ Kraftverlauf über der Zeit in N

$S_H$ Steifigkeit in N/m

$\eta_H$ eine der Viskosität verwandte Größe in N·s/m

$\ell_o$ statische Auslenkung in mm

$\ell_a$ dynamische Auslenkung in mm.

Die Steifigkeit kann dabei sowohl über die gemessene Kraft als auch über den gemessenen Weg in Abhängigkeit der eingebrachten Kraft oder besser in Abhängigkeit des Weges des Meßkörpers bestimmt werden. Durch diese doppelte Bestimmung kann die Qualität der Meßergebnisse weiter verbessert werden.

Die Reproduzierbarkeit der erhaltenen Werte wird zusätzlich dadurch gewährleistet, daß aufgrund der Geräteabstimmung, insbesondere hohe Steifigkeit und geringe bewegte Masse durch Piezobauweise, die Messungen weit unterhalb der Eigenresonanz des Gerätes erfolgen.

**Patentansprüche**

1. Vorrichtung zur Messung viskoelastischer Eigenschaften von Körpern, insbesondere von Körpern aus elastomerem Material wie Gummi, mit einem Meßkörper (2), welcher einen auf einen Probekörper aufsetzbaren Meßkopf (3) aufweist und in einem frei beweglichen Gehäuse (1) mit einer Öffnung (4) für den Durchtritt des Meßkopfes (3) und einer auf den Probekörper aufsetzbaren Anschlagfläche (5) verschiebbar gelagert ist, mit Mitteln (6) zum Hin- und Herbewegen des Meßkörpers (2) und Mitteln (7, 24) zum Messen des Verschiebeweges des Meßkopfes (3) und/oder der auf den Meßkopf (3) wirkenden Kraft während des Hin- und Herbewegens des Meßkörpers (2),
**dadurch gekennzeichnet,**
**daß** zum Hin- und Herbewegen des Meßkörpers (2) ein Piezoelement (6) vorgesehen ist, und daß der Meßkopf als Eindringkörper (3) ausgebildet ist, welcher entsprechend einer gewünschten Eindringtiefe (a) in Nullstellung über die Anschlagfläche (5) des Gehäuses (1) übersteht.

2. Vorrichtung nach Anspruch 1,
**dadurch gekennzeichnet,**
**daß** der Eindringkörper gemäß DIN 53505 zur Messung von Probekörpern aus elastomerem Material, insbesondere als Shore-A-Eindringkörper ausgebildet ist.

3. Vorrichtung nach Anspruch 1 oder 2,
**dadurch gekennzeichnet,**
**daß** die Eindringtiefe (a) in Nullstellung so gewählt ist, daß die Auslenkung des Eindringkörpers (3) in einem Bereich zumindest annähernd linearen Kraft-Weg-Verhältnisses liegt.

4. Vorrichtung nach Anspruch 3,
**dadurch gekennzeichnet,**
**daß** die Eindringtiefe (a) in Nullstellung zur Messung eines Körpers aus elastomerem Material ca. 100 bis 500 µm beträgt.

5. Vorrichtung nach einem der vorhergehenden Ansprüche,
**dadurch gekennzeichnet,**
**daß** die Auslenkung des Eindringkörpers (3) so klein gewählt ist,
**daß** sich der Probekörper annähernd linear verhält.

6. Vorrichtung nach Anspruch 5,
**dadurch gekennzeichnet,**
**daß** die Auslenkung des Eindringkörpers (3) zur Messung eines Körpers aus elastomerem Material ca. 5 bis ca. 25 µm beträgt.

7. Vorrichtung nach einem der vorhergehenden Ansprüche,
**dadurch gekennzeichnet,**
**daß** die Auslenkung des Eindringkörpers (3) während der Messung über den Auslenkweg festgelegt ist.

8. Vorrichtung nach Anspruch 7,
**dadurch gekennzeichnet,**
**daß** das Piezoelement (6) in einem Regelkreis mit einer Wegmeßeinrichtung (24) angeordnet ist.

9. Vorrichtung nach einem der vorhergehenden Ansprüche,
**dadurch gekennzeichnet,**
**daß** die Frequenz der Hin- und Herbewegung des Meßkopfes (3) wählbar ist.

10. Vorrichtung nach einem der vorhergehenden Ansprüche,
**dadurch gekennzeichnet,**
**daß** die Frequenz der Hin- und Herbewegung des Meßkopfes (3) ca. 1 Hz bis ca. 15 Hz, insbesondere ca. 10 Hz, beträgt.

11. Vorrichtung nach einem der vorhergehenden Ansprüche,
**dadurch gekennzeichnet,**
**daß** Mittel (25) zur Bestimmung der Temperatur des Probekörpers vorgesehen sind.

12. Vorrichtung nach einem der vorhergehenden Ansprüche,
**dadurch gekennzeichnet,**
**daß** Mittel (14) vorgesehen sind, durch welcher sicherstellbar ist,
**daß** die Anschlagfläche (5) während des Meßvorgangs fest auf dem Probekörper aufsitzt.

13. Vorrichtung nach Anspruch 12,
**dadurch gekennzeichnet,**
**daß** die Anschlagfläche (5) als Standfläche definierter Größe ausgebildet ist.

14. Vorrichtung nach einem der Ansprüche 1 bis 12,
**dadurch gekennzeichnet,**
**daß** ein Drucksensor (14) vorgesehen ist, welcher ein Anpressen der Anschlagfläche (5) an den Probekörper detektiert.

15. Vorrichtung nach Anspruch 14,
**dadurch gekennzeichnet,**
**daß** eine zwischen zwei einander zugewandten ebenen Flächen zweier Teile.(11, 15) des Gehäuses (1) eingespannte ebene Druckscheibe (14) vorgesehen ist.

16. Vorrichtung nach einem der vorhergehenden Ansprüche,

**dadurch gekennzeichnet,**
**daß** Anzeigemittel (27) vorgesehen sind, welche ein festgestelltes Anliegen der Anschlagfläche (5) am Probekörper anzeigen.

17. Vorrichtung nach einem der vorhergehenden Ansprüche,
**dadurch gekennzeichnet,**
**daß** Mittel (17) vorgesehen sind, welche bewirken, daß eine Messung nur bei festgestelltem Anliegen der Anschlagfläche (5) am Probekörper ausgeführt oder verwendet wird.

**Claims**

1. Apparatus for the measurement of viscoelastic characteristics of sample bodies, in particular of sample bodies of elastomeric materials, such as rubber, comprising a measurement body (2), which has a measuring head (3) for placement onto a sample body and is displaceably mounted in a freely movable housing (1) having an opening (4) for the passage of the measuring head (3) and an abutment surface (5) for placement onto the sample body, with means (6) for producing to and fro movement of the measurement body (2) and means (7, 24) for measurement of a path of displacement of the measurement head (3) and/or of the force acting on the measurement head (3) during the to and fro movement of the measurement body (2), **caracterized in that** a piezoelement (6) is provided for the to and fro movement of the measurement body (2) and that the measurement head is formed as a penetration body (3) which in the zero position projects beyond the abutment surface (5) of the housing (1) in accordance with a desired depth of penetration (a).

2. Apparatus in accordance with claim 1, **caracterized in that** the penetration body is formed in accordance with Deutsche Industrie Norm 53505 for the measurement of test bodies of elastomeric material, particularly as a Shore-A penetration bodies.

3. Apparatus in accordance with claim 1 or 2, **caracterized in that** the depth of penetration (a) in the zero position is selected so that displacement of the penetration body (3) lies in a region of approximately linear force-displacement ratio.

4. Apparatus in accordance with claim 3, **caracterized in that** the depth of penetration (a) in the zero position for the measurement of a body of elastomeric material amounts to ca. 100 to 500 μm.

5. Apparatus in accordance with one of the preceding claims, **caracterized in that** the displacement of the penetration body (3) is selected to be so small that the sample body behaves approximately linearly.

6. Apparatus in accordance with claim 5, **caracterized in that** the displacement of the penetration body (3) for the measurement of the body of elastomeric material amounts to ca. 5 to ca. 25 μm.

7. Apparatus in accordance with one of the preceding claims, **caracterized in that** the displacement of the penetration body (3) during the measurement is determined via the path of deflection.

8. Apparatus in accordance with claim 7, **caracterized in that** the piezoelement (6) is arranged in a regulating circuit with a path measuring device (24).

9. Apparatus in accordance with one of the preceding claims, **caracterized in that** the frequency of the to and fro movement of the measurement head (3) is selectable.

10. Apparatus in accordance with one of the preceding claims, **caracterized in that** the frequency of the to and fro movement of the measurement head (3) amounts to ca. 1 Hz to ca. 15 Hz, particularly to 10 Hz..

11. Apparatus in accordance with one of the preceding claims, **caracterized in that** apparatuses (25) for determining the temperature of the sample body are provided.

12. Apparatus in accordance with the preceding claims, **caracterized in that** apparatuses (14) are provided for ensuring that the abutment surface (5) sits firmly on the sample body during a measurement procedure.

13. Apparatus in accordance with claim 12, caracterized in that the abutment surface (5) is formed as a standing surface of defined size.

14. Apparatus in accordance with one of the claims 1 to 12, one pressure sensor (14) is provided for detecting a pressing contact of the abutment surface (5) at the sample body.

15. Apparatus in accordance with claim 14, **caracterized in that** a planar pressure disk (14), clamped between two confronting flat surfaces of two parts (11, 15) of the housing (1), is provided.

16. Apparatus in accordance with one of the preceding claims, **caracterized in that** indicator means (27) are provided for indicating a detected contact of the abutment surface (5) on the sample body.

17. Apparatus in accordance with one of the preceding claims, **caracterized in that** means (17) are provided for causing a measurement to be carried out or used only when contact of the abutment surface (5) on the sample body is detected.

## Revendications

1. Appareil de mesurage des propriétés viscoélastiques d'échantillons, en particulier d'échantillons en élastomère, tels que le caoutchouc, qui comprend un corps de mesurage (2), possédant une tête de mesurage (3) pour une installation sur un échantillon, et qui est monté de façon à pouvoir se déplacer dans un logement libre mobile (1) présentant une ouverture (4) pour le passage de la tête de mesurage (3), et une surface de butée (5) pour une installation sur l'échantillon, avec des moyens (6) afin de produire un mouvement de va-et-vient du corps de mesurage (2), et des moyens (7, 24) de mesurage de la voie de la tête de mesurage (3) en déplacement et/ou de la force qui agit sur la tête de mesurage (3) pendant le mouvement de va-et-vient du corps de mesurage (2), **caractérisé en ce qu'**un piézoélément (6) est prévu pour le mouvement de va-et-vient du corps de mesurage (2), et que la tête de mesurage est formée comme un pénétrateur (3), qui en position zéro se projette hors de la surface de butée (5) du logement (1) selon une profondeur de pénétration (a) désirée.

2. Appareil selon la revendication 1, **caractérisé en ce que** le pénétrateur est formé selon les normes de l'Institut allemand DIN 53505 pour le mesurage des corps de tests en élastomère, en particulier comme un pénétrateur Shore-A.

3. Appareil selon la revendication 1 ou 2, **caractérisé en ce que** la profondeur de pénétration (a) dans la position zéro est sélectionnée de telle manière, que le déplacement du pénétrateur (3) se situe dans une région ayant une proportion de force-déplacement plus ou moins linéaire.

4. Appareil selon la revendication 3, **caractérisé en ce que** la profondeur de pénétration (a) dans la position zéro, pour le mesurage des quantités de matériau élastomère présentes dans un corps se situe entre approximativement 100 et 500 μm.

5. Appareil selon l'une des revendications précédentes, **caractérisée en ce qu'**on a décidé de réduire le déplacement du pénétrateur (3) de telle manière, que l'échantillon se comporte plus ou moins de façon linéaire.

6. Appareil selon la revendication 5, **caractérisé en**

**ce que** le déplacement du pénétrateur (3) pour le mesurage du corps en matériau élastomère présente des quantités comprises entre approximativement 5 et 25 µm.

7. Appareil selon l'une des revendications précédentes, **caractérisé en ce que** le déplacement du pénétrateur (3) pendant le mesurage est déterminé par la voie de déflexion.

8. Appareil selon la revendication 7, **caractérisé en ce que** le piézoélément (6) est disposé dans un circuit de réglage avec un dispositif de mesurage de la voie (24).

9. Appareil selon l'une des revendications précédentes, **caractérisé en ce que** l'on peut sélectionner la fréquence du mouvement de va-et-vient de la tête de mesurage (3).

10. Appareil selon l'une des revendications précédentes, **caractérisé en ce que** la fréquence du mouvement de va-et-vient de la tête de mesurage (3) est présente dans des quantités comprises entre approximativement 1 Hz et15 Hz, et en particulier de 10 Hz.

11. Appareil selon l'une des revendications précédentes, **caractérisé en ce que** des appareils (25) sont prévus pour déterminer la température de l'échantillon.

12. Appareil selon les revendications précédentes, **caractérisé en ce que** ces appareils (14) sont prévus pour assurer l'installation de façon solide de la surface de butée (5) sur l'échantillon lors d'un processus de mesurage.

13. Appareil selon la revendication 12, **caractérisé en ce que** la surface de butée (5) est formée comme une surface en station debout de dimensions définies.

14. Appareil selon l'une des revendications 1 à 12, un capteur de pression (14) est proportionné pour détecter un contact de pression de la surface de butée (5) sur l'échantillon.

15. Appareil selon la revendication 14, **caractérisé en ce que** l'on prévoit un disque de pression plat (14), coincé entre deux surfaces plates opposées des deux parties (11, 15) du logement (1).

16. Appareil selon l'une des revendications précédentes, **caractérisé en ce que** des moyens d'indication (27) sont prévus pour indiquer un contact détecté de la surface de butée (5) avec l'échantillon.

17. Appareil selon l'une des revendications précédentes, **caractérisé en ce que** lesdits moyens (17) sont prévus pour fournir un mesurage qui doit être réalisé ou utilisé, uniquement si le contact de la surface de butée (5) avec l'échantillon a été détecté.

# FIG. 1

# FIG. 2

# FIG. 3

EP 0 900 368 B1